(19) Europäisches Patentamt
European Patent Office
Office européen des brevets

(11) Numéro de publication : 0 340 055 B1

# (12) FASCICULE DE BREVET EUROPEEN

(45) Date de publication du fascicule du brevet : **10.03.93 Bulletin 93/10**

(51) Int. Cl.$^5$ : **C07D 401/04**, A61K 31/47

(21) Numéro de dépôt : **89400873.9**

(22) Date de dépôt : **29.03.89**

## (54) Dérivés de l'acide 7-(1-imidazolyl)-1,4-dihydro-4-oxo-6,8-difluoro-1-cyclopropyl-3-quinoléincarboxylique, leur préparation et leur application en tant que médicaments.

Le dossier contient des informations techniques présentées postérieurement au dépôt de la demande et ne figurant pas dans le présent fascicule.

(30) Priorité : **31.03.88 FR 8804289**

(43) Date de publication de la demande : **02.11.89 Bulletin 89/44**

(45) Mention de la délivrance du brevet : **10.03.93 Bulletin 93/10**

(84) Etats contractants désignés : **AT BE CH DE FR GB GR IT LI LU NL SE**

(56) Documents cités :
**EP-A- 0 115 049**
**EP-A- 0 203 488**

(56) Documents cités :
**JOURNAL OF MEDICINAL CHEMISTRY, vol. 30, no. 12, décembre 1987, pages 2163-2169, American Chemical Society; T. UNO et al.: "Synthesis of antimicrobial agents. 1. Syntheses and antibacterial activities of 7-(Azole substituted)quinolones"**

(73) Titulaire : **LABORATORIOS DEL DR. ESTEVE, S.A.
Av. Mare de Deu de Montserrat, 221
E-08026 Barcelona (ES)**

(72) Inventeur : **Pares Corominas, Juan
Padilla 349 3o 3a
E-08025 Barcelone (ES)**
Inventeur : **Colombo Pinol, Augusto
Av. Chile 36 4o 1a
E-08032 Barcelone (ES)**
Inventeur : **Frigola Constansa, Jordi
Av. Diagonal 299 at. 1a
E-08013 Barcelone (ES)**

(74) Mandataire : **Ahner, Francis et al
CABINET REGIMBEAU, 26, avenue Kléber
F-75116 Paris (FR)**

EP 0 340 055 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à de nouveaux dérivés des acides 1,4-dihydro-4-oxoquinoléine-3-carboxyliques substitués en position 7 par un radical 1-imidazole substitué.

Les imidazoles liés à la position 7 des acides 1,4-dihydro-4-oxoquinoléine-3-carboxyliques ont été très peu étudiées. Autant que l'on sache, il n'existe qu'un petit nombre de publications dans la littérature scientifique qui se rapportent à ce genre de composés.

Dans DE-A- 3519286, on décrit l'acide 1-cyclopropyl-6,8-difluoro-1,4-dihydro-7-(1-imidazolyl)-4-oxoquinoléine-3-carboxylique.

T. Uno et coll. ont décrit, dans J. Med. Chem. 1984, 30, 2163 l'acide 1-éthyl-6-fluoro-7-(1-imidazolyl)-4-oxoquinoléine-3-carboxylique et ses dérivés avec des imidazoles substitués tels le 2-méthyl et les 4-méthyl, nitro, bromo, méthylcarboxylate, diméthylcarboxamido, formyl, méthanol, diméthylaminométhyl et hydroxyiminométhyl.

L'invention concerne des composés représentés par la formule (I) ci-après,

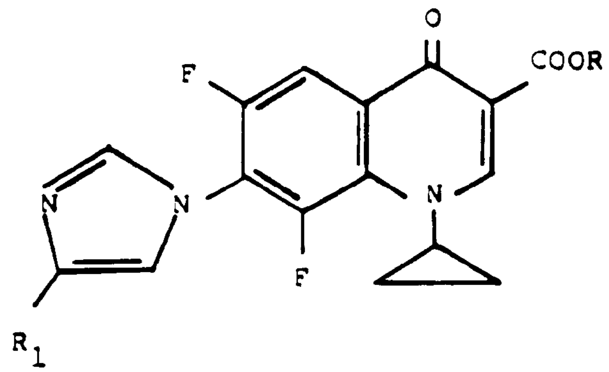

(I)

où R représente un atome d'hydrogène, un radical alkyle $C_1$-$C_4$ ou un radical phényle, et $R_1$ un radical alkyle en $C_1$-$C_4$ ou un radical phényle.

La présente invention se rapporte également aux sels physiologiquement acceptables des composés de formule générale I.

L'invention concerne également une composition pharmaceutique contenant un composé de formule (I) ou un de ses sels pharmaceutiquement acceptable en une quantité antimicrobienne efficace.

De plus, l'invention concerne des procédés pour préparer les composés de formule (I) et leurs sels pharmaceutiquement acceptables.

Dans l'ensemble de la description, le terme alkyle inférieur désignera des radicaux hydrocarbonés linéaires ou ramifiés comprenant de préférence de 1 à 4 atomes de carbone.

On peut préparer les composés de l'invention représentés par la formule (I) selon divers procédés connus.

Par exemple, un procédé consiste à faire réagir un composé hétérocyclique de formule (II) :

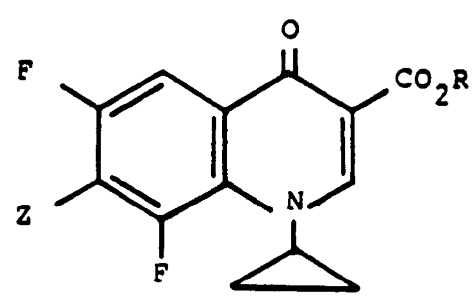

(II)

où R a la même signification que précédemment, et Z représente un atome d'halogène ; avec un imidazole représenté par la formule (III) :

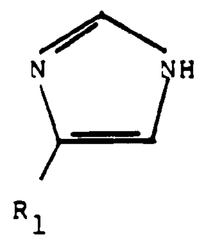

(III)

où $R_1$ a la même signification que précédemment.

On peut effectuer la réaction dans un grand nombre de solvants. On peut citer comme exemples, des alcools inférieurs tels que l'éthanol, l'isopropanol, etc., des éthers tels que le tétrahydrofuranne, le dioxanne, le diglyme, etc., des nitriles tels que l'acétonitrile, la pyridine, le diméthylsulfoxyde, le diméthylformamide et l'hexaméthylphosphorotriamide.

On peut effectuer la réaction ci-dessus en présence d'un accepteur d'acide, en une quantité au moins approximativement comprise entre 1 et 2 moles par mole de composé de formule (II). On peut citer comme exemples d'accepteurs d'acide appropriés, des hydroxydes de métal alcalin, des carbonates minéraux et des amines tertiaires telles que la triéthylamine.

Les composés de formule (II) que l'on peut utiliser comme matières de départ pour préparer les composés de l'invention représentés par la formule (I) sont des composés connus, comme décrits par exemple dans H. Koga, A. Itoh, S. Murayama, S. Suzue et T. Irikura, J. Med. Chem., 1980, 23, 1358.

D'autre part, les composés de formule (III) qui constituent d'autres matières de départ pour préparer les composés de l'invention représentés par la formule (I) sont connus, ou sont synthétisés comme décrit par exemple dans divers articles : Bulavain et coll. J. Chem. Soc. 1922, 947, Kunitake, T., et coll. Bull Chem. Soc. Jpn. 1975, 48, 1304 ; Batterby et coll. J. Chem. Soc. 1970, 49, Turner R.A. et coll. J.A.C.S. 1949, 71, 2801 ; Hulball, W. et coll. J. Chem. Soc. 1928, 21, ou bien ils sont commerciaux.

On peut effectuer la réaction ci-dessus à pression atmosphérique ou à une pression d'environ 1 à 15 $kg/cm^2$, et à une température d'environ 10 à 50°C pendant une durée d'environ 1 à 5 jours et après, à une température d'environ 50 à 150°C pendant une durée d'environ 2 à 72 heures.

Dans les exemples suivants, on indiquera la préparation de nouveaux dérivée selon l'invention. On décrira également quelques formes d'emploi typiques pour les différents domaines d'application.

Les exemples ci-après sont donnés à simple titre d'illustration.

Exemple 1

Préparation de l'acide 1-cyclopropyl-6,8-difluoro-7-(4-phénylimidazo-1-yl)-1,4-dihydro-4-oxo-3-quinoléincarboxylique.

On chauffe au reflux pendant 3 heures un mélange de 2,8 g (10 mmoles) d'acide 1-cyclopropyl-6,7,8-trifluoro-1,4-dihydro-4-oxo-3-quinoléincarboxylique, 4,3 g (30 mmoles) de 4-phénylimidazole, 20 ml de triéthylamine et 100 ml de DMSO. On laisse refroidir, on verse sur un mélange eau/glace, on ajuste à pH 5 et on obtient un précipité que l'on filtre et on lave à l'eau. On sèche le solide sous vide et on obtient 2,3 g d'acide 1-cyclopropyl-6,8-difluoro-7-(4-phénylimidazo-1-yl)-1,4-dihydro-4-oxo-3-quinoléincarboxylique, de point de fusion 286-288°C. Données spectroscopiques 1H RMN [DMSO-6d], δ ; 1, 29 (b, 4H) ; 4,10 (m,1H) ; 7,33 (m, 3H) ; 7,90 (m, 5H) , 8,80 (s, 1H).

IR : (KBr) : 3115, 3050, 1735, 1470 $cm^{-1}$.

Exemple 2

Préparation de l'acide 1-cyclopropyl-6,8-difluoro-7-(4-méthylimidazo-1-yl)-1,4-dihydro-4-oxo-3-quinoléincarboxylique.

On chauffe au reflux un mélange de 2,8 g (10 mM) d'acide 1-cyclopropyl-6,7,8-trifluoro-1,4-dihydro-4-oxo-3-quinoléincarboxylique, 2,1 g (25 mM) de 4-méthylimidazole, 30 ml de triéthylamine dans 150 ml d'acétonitrile pendant 5 heures. On concentre sous pression réduite et on filtre le précipité. On reprend le résidu dans 50 ml d'eau, on ajuste à pH 5 et on filtre et lave le précipité. On peut recristalliser dans l'acétonitrile.

On obtient ainsi 2,3 g de l'acide 1-cyclopropyl-6,8-difluoro-7-(4-méthylimidazo-1-yl)-1,4-dihydro-4-oxo-3-quinoléincarboxylique, de point de fusion 215-223°C.

Données spectroscopiques : 1H RMN [DMSO-6d], δ; 1,29 (b, 4H) ; 2,23 (s, 3H) ; 3,27 (b, $H_2O$) ; 4,15 (m, 1H) ; 7,16 (s, 1H) ; 7,85 (S, 1H) ; 8,10 (d, 1H) ; 8,8 (s, 1H).

IR : (KBr) : 3050, 1735, 1615 $cm^{-1}$.

Activité pharmacologique antimicrobienne

(G.L. Daguet et Y.A. Chabbect, Techniques en bactériologie, Vol. 3, Flammarion Médecine-Sciences, Paris, 1972 et W.B. Hugo et A.D. Rusell, Pharmaceutical Microbiology, Blackwell Scientific Publications, London, (1977)).

- Milieu de culture et solvant :

Agar d'antibiotiques n° 1 (Oxoid OM 327)
Bouillon de triptone-soja (Oxoid OM 129)
Solution physiologique Ringer 1/4 (Oxoid BR 52)
Agar Dextrose (BBL-11165)
NaOH 0,1N

- Microorganismes :

"Bacilus subtilis" ATCC 6633
"Citrobacter freundii" ATCC 11606
"Enterobacter aerogenes" ATCC 15038
"Enterobacter cloacae" ATCC 23355
"Escherichia coli" ATCC 10536
"Escherichia coli" ATCC 23559
"Klebsiella pneumoniae" ATCC 10031
"Proteus mirabilis" ATCC 4675
"Proteus vulgaris" ATCC 8427
"Pseudomonas aeruginosa" ATCC 9721
"Pseudomonas aeruginosa" ATCC 10145
"Salmonella tiphymurium" ATCC 14028
"Serratia marcescens ATCC 13880
"Shigella flexnerii" ATCC 12022
"Staphylococcus aureus" ATCC 25178
"Streptococcus faecalis" ATCC 10541

-Préparation des inoculations

Chacun des microorganismes est ensemencé par strie dans des tubes d'Agar d'antibiotiques n° 1, et mis en incubation pendant 20 heures à 37°C. Ensuite, on prend une anse de culture, on ensemence dans un bouillon de Triptone-soja et on incube pendant 20 heures à 37°C. On dilue à 1/4 la culture obtenue avec une solution physiologique Ringer, de façon à obtenir une suspension normalisée de $10^7$-$10^9$ ufc/ml pour chaque organisme.

- Préparation du milieu contenant les dérivés de formule générale I

A partir d'une solution de 1000 μg/ml dans NaOH 0,1N, chaque produit est dilué dans l'Agar Dextrose (préalablement fondu et maintenu à 50°C) par dilutions successives de façon à obtenir les concentrations suivantes : 64 - 32 - 16 - 8 - 4 - 2 - 1 - 0,5 - 0,25 - 0,125 μg de dérivé/ml du milieu.

Postérieurement chaque concentration de chaque produit est répartie dans des boîtes de pétri de 10 cm de diamètre, à raison de 10 ml de milieu par boîte et autant de boîtes que de microorganismes à tester.

Une fois le milieu refroidi, les boîtes sont ensemencées avec les inoculations à raison de 0,4 ml d'inoculation par boîte. On les étend avec une anse de Driglasky et on recueille le surnageant. Les boîtes ensemencées sont incubées à 37°C pendant 20 heures.

Résultats

Les résultats obtenus sont décrits dans le tableau I. Les composés des exemples ont une activité "in vitro" supérieure à celle de l'acide pipémidique, tant à l'égard des Enterobacteriaceas, (pseudomonas aeruginosa) que des coques Gram-positifs. Les composés des exemples présentent une activité supérieure, face aux coques Gram-positifs et aux microorganismes Gram-négatifs, à celle de l'acide pipémidique.

TABLEAU I

CMI "in vitro" comparée à l'acide pipémidique.

Les concentrations sont données en µg/ml.

| | Composé de l'exemple 2 | Acide pipémidique | Composé de l'exemple 1 |
|---|---|---|---|
| Bacilus subtilis ATCC 6633 | ≤0,03 | 8 | ≤0,03 |
| Citrobacter freundii ATCC 11606 | 0,12 | 4 | 2 |
| Enterobacter aerogenes ATCC 15038 | 0,25 | 32 | 2 |
| Enterobacter chloacae ATCC 23335 | 0,12 | 8 | 2 |
| Escherichia coli ATCC 10536 | 0,25 | 2 | 2 |
| Escherichia coli ATCC 23559 | 0,12 | 16 | 1,0 |
| Klebsiella pneumoniae ATCC 10031 | ≤0,003 | 2 | 0,25 |
| Proteus mirabilis ATCC 4675 | 0,50 | 16 | 4 |
| Proteus vulgaris ATCC 8427 | 0,12 | 8 | 1 |
| Pseudomonas aeruginosa ATCC 9721 | 2,0 | 32 | 4,0 |
| Pseudomonas aeruginosa ATCC 10145 | 4,0 | 32 | 16 |
| Salmonella tiphymurium ATCC 14028 | 0,25 | 4 | 2 |
| Serratia marcescens ATCC 13880 | 0,50 | 16 | 4 |
| Shigella flexnerii ATCC 12022 | 0,25 | 4 | 0,5 |
| Staphylococcus aureus ATCC 25178 | 0,06 | 64 | 0,06 |
| Streptococcus faecalis ATCC 10541 | 0,25 | 64 | 0,12 |

Compte tenu de leurs bonnes propriétés pharmacologiques, les dérivés de formule générale I sont donc susceptibles d'être utilisés en médecine humaine et/ou vétérinaire, pour le traitement des infections aiguës, chroniques et récidivantes systémiques ou localisées, causées par des microorganismes Gram-positifs et Gram-négatifs sensibles aux produits objet de la présente invention, dans le tractus gastro-intestinal ou génito-urinaire, l'appareil respiratoire, la peau et les tissus mous, ainsi que les infections neurologiques et odontostomatologiques.

En thérapeutique humaine, la dose proposée des dérivés de la présente invention est environ comprise entre 400 et 1200 mg/jour pour un adulte, par exemple administrée sous la forme de comprimés ou de gélules. Cette posologie peut toutefois varier en fonction de la gravité de l'affection.

On indiquera ci-après, à titre d'exemples, deux formes galéniques particulières des dérivés, objet de la présente invention.

| Exemple de formule par comprimé | |
|---|---|
| Composé de l'exemple 2 | 0,400 g |
| Carboxyméthylamidon | 0,018 g |
| Polyvinylpyrrolidone K29-32 | 0,030 g |
| Cellulose microcristalline | 0,146 g |
| Silice colloïdale | 0,003 g |
| Stéarate de magnésium | 0,003 g |
| | 0,600 g |

| Exemple de formule par gélule | |
|---|---|
| Composé de l'exemple 2 | 0,400 g |
| Cellulose microcristalline | 0,0356 g |
| Silice colloïdale | 0,0022 g |
| Stéarate de magnésium | 0,0022 g |
| | 0,440 g |

**Revendications**

1. Nouveaux composés hétérocycliques caractérisés en ce qu'ils répondent à la formule (I)

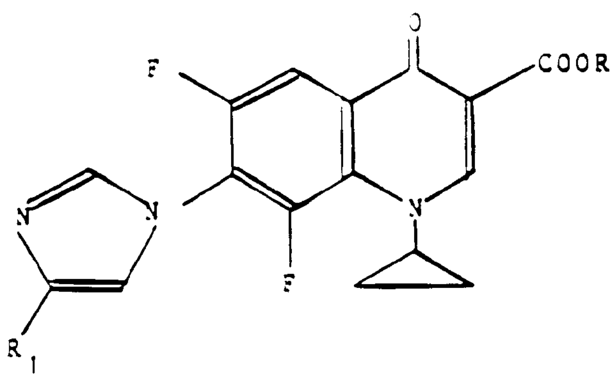

(I)

où :

R représente un atome d'hydrogène, un radical alkyle en $C_1$-$C_4$ ou un radical phényle et

$R_1$ représente un radical alkyle en $C_1$-$C_4$ ou un radical phènyle ainsi que leur sels physiologiquement acceptables.

2. Composé répondant à la formule générale I, selon la revendication 1, choisi parmi : l'acide 1-cyclopropyl-6,8-difluoro-7-(4-phénylimidazo-1-yl)-1,4-dihydro-4-oxo-3-quinoléincarboxylique ; et l'acide 1-cyclopropyl-6,8-difluoro-7-(4-méthylimidazo-1-yl)-1,4-dihydro-4-oxo-3-quinoléincarboxylique.

**3.** Procédé de préparation des dérivés de formule (I), selon l'une des revendications 1 et 2, caractérisé en ce qu'il consiste à faire réagir un composé hétérocyclique de formule (II)

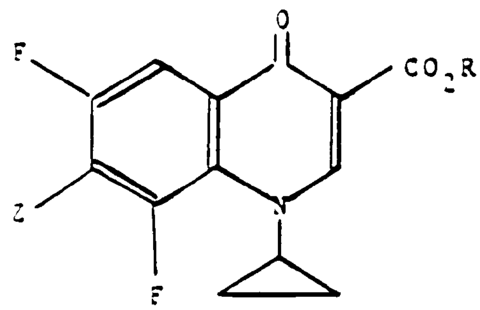

(II)

où :

R a la même signification que précédemment, et

Z représente un atome d'halogène ; avec un imidazole répondant à la formule (III)

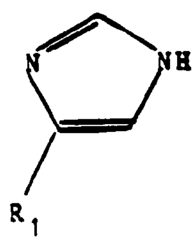

(III)

où :

$R_1$ a la même signification que précédemment.

**4.** A titre de médicaments, les composés de formule générale (I) et leurs sels thérapeutiquement acceptables selon l'une des revendications 1 et 2, en particulier comme antibactériens.

**5.** Compositions pharmaceutiques, caractérisées par le fait qu'elles contiennent, outre un support pharmaceutiquement acceptable, au moins un composé de formule générale (I) ou l'un de leurs sels physiologiquement acceptables, selon l'une des revendications 1 et 2.

## Patentansprüche

**1.** Neue heterocyclische Verbindungen, dadurch gekennzeichnet, daß sie der allgemeinen Formel (I)

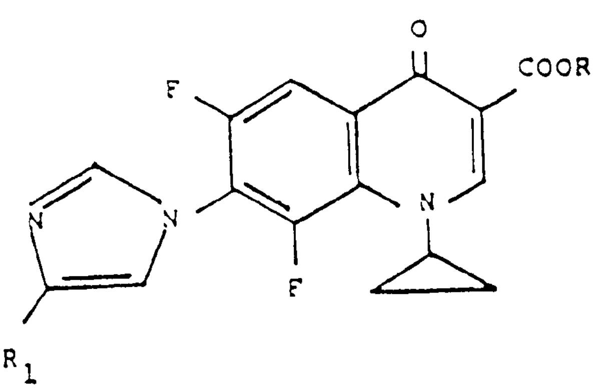

(I)

entsprechen, in der

R ein Wasserstoffatom, ein Alkyl-Radikal mit 1 bis 4 Kohlenstoffatomen oder ein Phenyl-Radikal darstellt, und

$R_1$ ein Alkyl-Radikal mit 1 bis 4 Kohlenstoffatomen oder ein Phenyl-Radikal bedeutet,
sowie ihre physiologisch akzeptablen Salze.

2. Verbindung der allgemeinen Formel (I) nach Anspruch 1, ausgewählt unter: 1-Cyclopropyl-6,8-difluor-7-(4-phenylimidazo-1-yl)-1,4-dihydro-4-oxo-3-chinolincarbonsäure und 1-Cyclopropyl-6,8-difluor-7-(methylimidazo-1-yl)-1,4-dihydro-4-oxo-3-chinolincarbonsäure.

3. Verfahren zur Herstellung der Derivate der Formel (I) nach einem der Ansprüche 1 und 2, dadurch gekennzeichnet, daß es darin besteht, eine heterocyclische Verbindung der Formel (II)

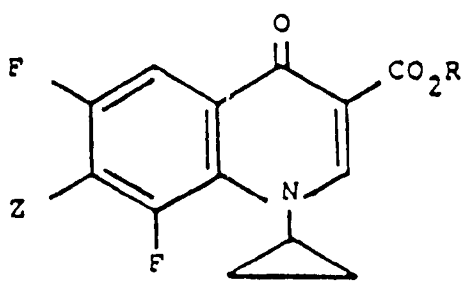

(II)

in der
R die gleiche Bedeutung wie vorstehend besitzt, und
Z ein Wasserstoffatom darstellt,
mit einem Imidazol der Formel (III)

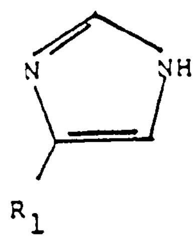

(III)

zur Reaktion zu bringen, in der $R_1$ die gleiche Bedeutung wie vorstehend besitzt.

4. Als Medikamente die Verbindungen der allgemeinen Formel (I) und ihre therapeutisch akzeptablen Salze, nach einem der Ansprüche 1 und 2, insbesondere als antibakterielle Mittel.

5. Pharmazeutische Zusammensetzungen, dadurch gekennzeichnet, daß sie außer einem pharmazeutisch akzeptablen Träger mindestens eine Verbindung der allgemeinen Formel (I) oder eines ihrer pharmazeutisch akzeptablen Salze, nach einem der Ansprüche 1 und 2, enthalten.

## Claims

1. Novel heterocyclic compounds, characterized in that they have formula (I)

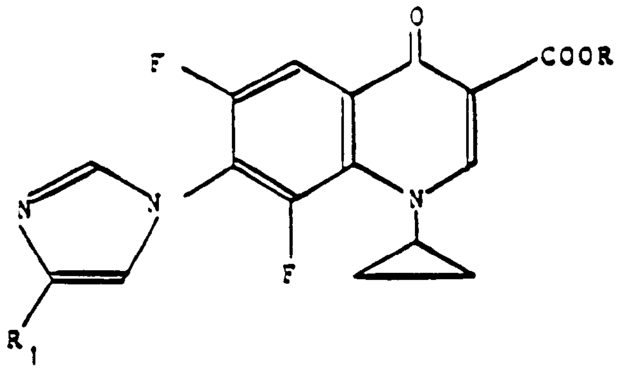

(I)

in which:

R represents a hydrogen atom, a $C_1$-$C_4$ alkyl radical or a phenyl radical, and

$R_1$ represents a $C_1$-$C_4$ alkyl radical or a phenyl radical,

and their physiologically acceptable salts.

2. A compound of general formula I according to Claim 1, selected from: 1-cyclopropyl-6,8-difluoro-7-(4-phenylimidazol-1-yl)-1,4-dihydro-4-oxoquinoline-3-carboxylic acid and 1-cyclopropyl-6,8-difluoro-7-(4-methylimidazol-1-yl)-1,4-dihydro-4-oxoquinoline-3-carboxylic acid.

3. A process for the preparation of the derivatives of formula (I) according to Claim 1 or Claim 2, characterized in that it consists in reacting a heterocyclic compound of formula (II)

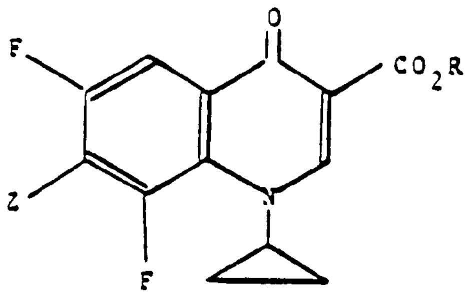

(II)

in which:

R is defined as above, and

Z represents a halogen atom,

with an imidazole of formula (III)

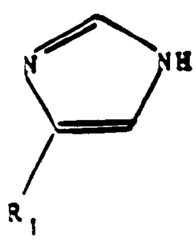

(III)

in which:

$R_1$ is defined as above.

4. As drugs, the compounds of general formula (I) and their therapeutically acceptable salts according to Claim 1 or Claim 2, in particular as antibacterial agents.

5. Pharmaceutical compositions, characterized in that they contain, in addition to a pharmaceutically acceptable excipient, at least one compound of general formula (I) or one of their physiologically acceptable salts according to Claim 1 or Claim 2.